# FASCICULE DE BREVET EUROPEEN

(11) **EP 2 139 414 B1**
(45) Date de publication et mention de la délivrance du brevet: **03.05.2017**
(21) Numéro de dépôt: 08805663.5
(22) Date de dépôt: 17.04.2008
(51) Int. Cl.: A61B 17/80

(54) **DISPOSITIF ORTHOPEDIQUE IMPLANTABLE CONSTITUE D'UNE STRUCTURE SUPPORT MUNIE D'AU MOINS UN ORIFICE ASSOCIE A UN ECROU, POUR LE PASSAGE D'UNE VIS DE FIXATION**
IMPLANTIERBARE ORTHOPÄDISCHE VORRICHTUNG AUS EINER TRÄGERSTRUKTUR MIT MINDESTENS EINER MIT EINER MUTTER AUSGESTATTETEN BOHRUNG ZUR HINDURCHFÜHRUNG EINER FIXIERSCHRAUBE
IMPLANTABLE ORTHOPAEDIC DEVICE COMPOSED OF A SUPPORT STRUCTURE HAVING AT LEAST ONE ORIFICE ASSOCIATED WITH A NUT, FOR THE PASSAGE OF A LOCKING SCREW

(30) Priorité: 17.04.2007 FR 0702777
(43) Date de publication de la demande: 06.01.2010
(73) Titulaire: D.L.P., 44690 La Haye Fouassiere (FR)
(72) Inventeur: DEROUET, Guillaume, F-44800 Saint-herblain (FR)
(74) Mandataire: Michelet, Alain
(86) Numéro de dépôt international: PCT/FR2008/050688
(87) Numéro de publication internationale: WO 2008/145902

(56) Documents cités:
- EP-A- 0 988 833
- EP-A- 1 488 754
- WO-A-01/15612
- WO-A-03/043513
- FR-A- 2 792 185
- FR-A- 2 867 962

## Description

La présente invention concerne un nouveau système d'implant orthopédique du type constitué d'une structure support munie d'au moins un orifice associé à un écrou, pour le passage d'une vis de fixation destinée à venir se fixer dans le matériau osseux de réception.

La plupart des implants orthopédiques, en particulier les implants d'ostéosynthèse, comprennent une structure support, souvent de type plaque, dans laquelle sont ménagés plusieurs orifices destinés au passage d'une vis de fixation apte à venir s'ancrer dans le matériau osseux de réception.
La forme générale et les dimensions de la plaque support sont adaptées aux contraintes d'implantation.

Certains implants se distinguent par la présence de moyens conférant à la vis de fixation une possibilité d'orientation angulaire par rapport à l'axe de son orifice de réception ; ils permettent au praticien de positionner au mieux la vis dans le matériau osseux de réception, notamment en fonction du site d'implantation et des contraintes spatiales rencontrées.

Des implants de ce genre, particulièrement intéressants, sont décrits dans le document WO-A-03/043513.
Ces implants orthopédiques sont du type comprenant une structure support munie d'au moins un orifice pour le passage d'une vis de fixation, et dont les contours délimitent un logement d'accueil d'un écrou muni d'un filetage interne destiné à coopérer avec un filetage complémentaire ménagé au niveau de la tête de vis.
L'écrou est bloqué en rotation dans son logement de réception de sorte à empêcher sa rotation autour de son axe (correspondant à l'axe de son filetage) ; il dispose encore d'au moins un degré de liberté dans le logement, selon un domaine d'inclinaison prédéterminé admissible, permettant ainsi une inclinaison de son axe par rapport à l'axe du logement de réception, pour autoriser un autocentrage de la vis et de l'écrou associé quelle que soit l'orientation admissible de l'axe de la vis par rapport à l'axe de l'orifice associé.
Dans ce genre d'implant, la tête de la vis est destinée à venir prendre appui d'un côté du support, et l'écrou est destiné à venir prendre appui de l'autre côté de ce support, cela de manière à permettre un serrage de la structure support entre ces deux éléments, en fin de serrage du corps de vis dans le matériau osseux de réception.
En pratique, les implants correspondants disposent de bonnes qualités de maintien en place et de verrouillage, tout en autorisant une possibilité intéressante d'orientation spatiale des vis de fixation par rapport à l'axe de leur orifice de réception.

Partant de ce genre d'implant, la demanderesse a développé une nouvelle structure simple et pratique, disposant là encore de bonnes qualités de blocage et de maintien en place, tout en autorisant une certaine possibilité d'orientation spatiale des vis de fixation au sein de leurs orifices de réception.
Les implants objets de la présente invention ont encore l'intérêt de pouvoir être très compacts et de présenter un encombrement réduit en épaisseur.

Pour cela, le dispositif orthopédique selon l'invention est du type comprenant :
- une structure support, délimitée par une face supérieure et par une face inférieure, cette dernière étant destinée à venir se positionner en regard du matériau osseux de réception, laquelle structure support est munie d'au moins un logement et d'au moins un orifice passant au travers dudit logement, ledit logement et ledit orifice ayant le même axe,
- au moins un écrou muni d'un filetage interne, lequel écrou est conformé pour être emprisonné au sein de l'un desdits logements, et
- au moins une vis de fixation composée d'une tête de vis et d'un corps de vis, laquelle tête de vis est munie d'un filetage apte à coopérer avec ledit filetage d'écrou, et lequel corps de vis est muni d'un filetage apte à coopérer avec le matériau osseux de réception,
lequel écrou est bloqué en rotation dans ledit logement de réception de sorte à empêcher sa mise en rotation autour de son axe, et lequel écrou comporte au moins un degré de liberté dans ledit logement de réception, selon un domaine d'inclinaison prédéterminé admissible, pour permettre une inclinaison de son axe par rapport à l'axe du logement de réception, ladite vis de fixation étant destinée à être vissée au sein de l'un desdits orifices et de son écrou associé, cela selon un angle sélectionné dans le domaine d'inclinaison admissible.
Conformément à l'invention, le logement de réception de l'écrou comporte une partie supérieure, située du côté de la face supérieure de la structure support, et une partie inférieure, située du côté de la face inférieure de ladite structure support, qui sont toutes deux en forme générale de tronc de sphère, concentriques l'une par rapport à l'autre. De plus, l'écrou comporte un contour périphérique en forme de tronc de sphère, complémentaire de la partie supérieure en tronc de sphère du logement de réception, et la tête de vis comporte une partie supérieure munie du filetage précité et une partie inférieure dont le contour a une forme générale en tronc de sphère, complémentaire de la partie inférieure en tronc de sphère du logement de réception.
Les parties supérieure et inférieure de la tête de vis sont destinées à coopérer, lors de la phase finale de vissage de la vis, respectivement avec le filetage complémentaire de l'écrou et avec la partie inférieure du logement de réception, cela de sorte à tendre à éloigner ledit écrou par rapport à la partie inférieure du logement de réception, pour conduire au verrouillage de la vis, par la poursuite du vissage, du fait d'un appui forcé du contour périphérique de l'écrou contre la partie supérieure complémentaire du logement de réception et, dans le même temps, d'un appui forcé de la partie inférieure de la tête de vis contre la partie inférieure complémentaire du logement.

Selon une autre particularité, les parties en tronc de sphère inférieure et/ou supérieure du logement de réception de l'écrou sont constituées par la surface centrale d'un clip rapporté, destiné à fermer partiellement ledit logement. L'utilisation de tels clips rapportés vise en particulier à faciliter la mise en place des écrous au sein de leurs logements de réception (mais aussi leur éventuel démontage), tout en assurant un maintien efficace de ces écrous en position.
Ce type de clip se présente avantageusement sous la forme d'un anneau ouvert, comportant une nervure d'encliquetage apte à venir se loger dans une gorge de forme appropriée ménagée dans le logement d'intégration de l'écrou.

Selon une forme de réalisation préférée, les parties en tronc de sphère supérieure et inférieure du logement de réception de l'écrou sont constituées, respectivement, par une surface monobloc de la structure support et par la surface centrale d'un clip rapporté.
Une telle structure permet d'utiliser le matériau osseux comme une surface d'appui assurant un maintien optimal du clip en position sur la structure support.

Selon d'autres caractéristiques, pouvant être prises en combinaison ou indépendamment les unes des autres :
- l'encombrement général de la tête de vis correspond au diamètre de l'orifice central de l'écrou, cet encombrement de la tête de vis étant encore inférieur à celui de la partie supérieure du logement de réception ;
- la hauteur de la tête de vis, entre son extrémité libre et l'extrémité inférieure de sa partie inférieure en tronc de sphère, est inférieure ou égale à la hauteur de l'orifice traversant associé ;
   ces caractéristiques structurelles contribuent chacune à optimiser l'aspect compact du dispositif, et en particulier participent à limiter l'encombrement lié à la tête de vis, cette dernière pouvant se loger entièrement dans l'épaisseur de la structure support ;
- le logement de réception de l'écrou comporte une partie supérieure en tronc de sphère dont le rayon est plus grand que celui de la partie inférieure en tronc de sphère ;
- les moyens pour bloquer l'écrou en rotation dans son logement d'accueil sont constitués d'au moins un relief ménagé sur l'une desdites pièces, coopérant avec une réservation adaptée ménagée sur l'autre desdites pièces ;
- le diamètre externe du filetage de corps de la vis est inférieur au diamètre externe du filetage de tête, et ce filetage de tête est composé de n filets, n étant supérieur ou égal à 2, décalés de 1/n tour, dont le pas correspond à celui du filetage de l'écrou et à celui dudit filetage de corps de vis. La pluralité de filets permet d'optimiser les caractéristiques de serrage de la tête de vis sur l'écrou.

L'invention sera encore illustrée, sans être aucunement limitée, par la description suivante d'une forme de réalisation particulière, donnée uniquement à titre d'exemple et représentée sur les dessins annexés suivants, dans lesquels :
- la figure 1 est une vue générale d'un dispositif orthopédique implantable conforme à l'invention, selon une perspective orientée du côté de sa surface supérieure, et dans laquelle la vis de fixation est convenablement montée au travers d'un orifice de réception d'une plaque support représentée schématiquement ;
- la figure 2 est encore une vue générale du dispositif orthopédique de la figure 1, selon une perspective orientée du côté de la face inférieure de sa plaque support (destinée à venir en appui contre la surface du matériau osseux de réception) ;
- la figure 3 montre le dispositif orthopédique conforme aux figures 1 et 2, selon un plan de coupe passant par l'axe de l'orifice traversant de la plaque support ;
- la figure 4 est une vue en perspective éclatée du dispositif orthopédique conforme aux figures 1 à 3, montrant en détail ses différents éléments constitutifs.

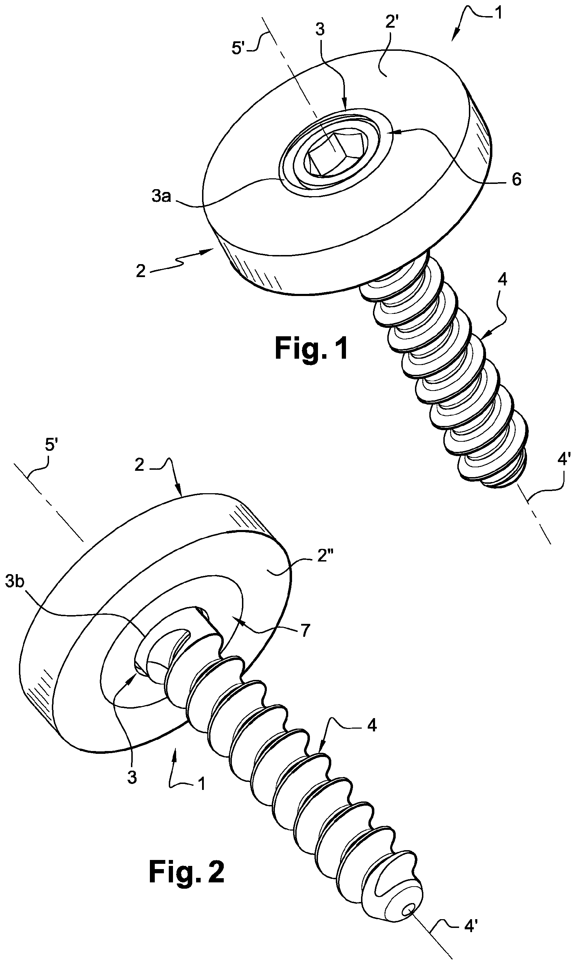

Le dispositif orthopédique implantable 1, représenté en perspective sur les figures 1 et 2, comprend principalement une structure support 2 (représentée ici schématiquement) dans laquelle est ménagé un orifice circulaire traversant 3 recevant une vis de fixation 4. Cette structure support 2 consiste par exemple en une plaque de 2 à 3 mm d'épaisseur délimitée par une face supérieure 2' et par une face inférieure 2". La vis 4 est destinée à être insérée dans l'orifice 3 du côté de la face supérieure 2' et elle en ressort par la face inférieure 2", cette dernière étant destinée à venir en appui contre le matériau osseux de réception (non représenté).

De manière générale, la plaque support 2 peut comporter plusieurs orifices traversants 3 convenablement agencés les uns par rapport aux autres.
Ce type d'implant orthopédique est en particulier destiné à être positionné sur un os fracturé (non représenté), par exemple une épiphyse de radius.

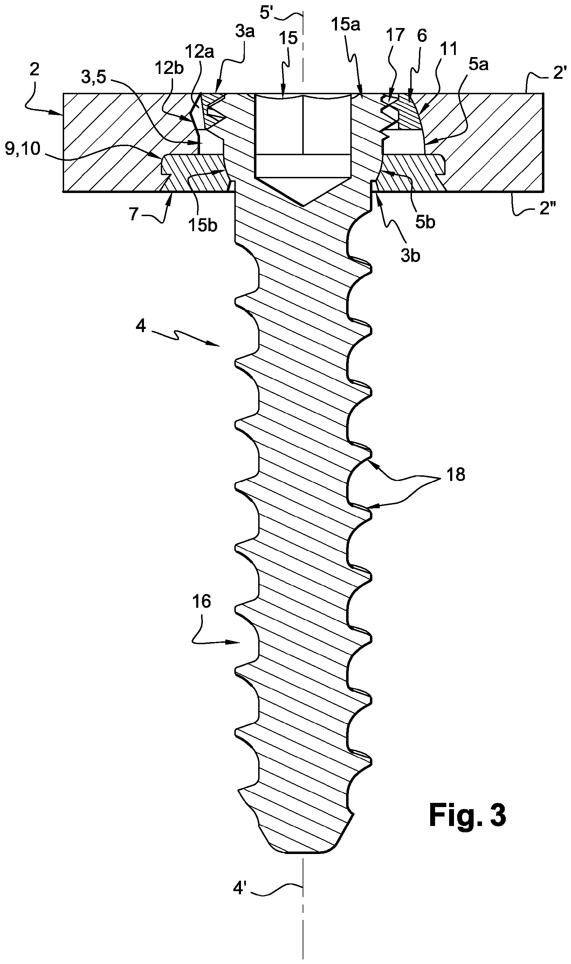

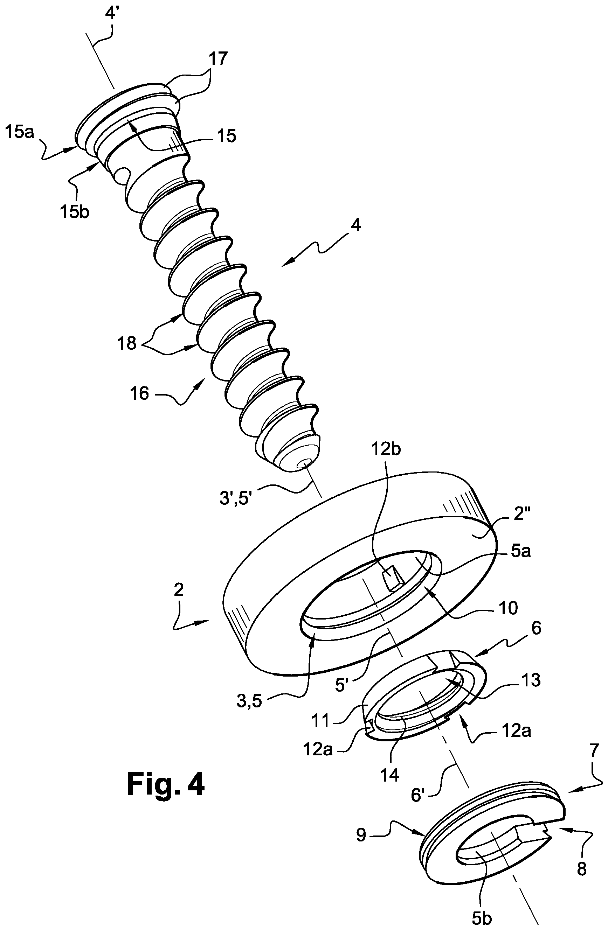

Sur les figures 3 et 4, on remarque que l'orifice traversant 3 s'étend au travers d'un logement 5 dans lequel est positionné et emprisonné un écrou 6 apte à coopérer avec un filetage complémentaire de la vis de fixation 4.
L'écrou 6 comporte un certain degré de liberté au sein de son logement de réception 5, selon un domaine d'inclinaison prédéterminé admissible, pour obtenir un verrouillage efficace de la vis de fixation 4 en fin de vissage dans le matériau osseux de réception, ceci quelle que soit l'orientation admissible de son axe 4' par rapport à l'axe 5' de l'orifice traversant 3 associé. En outre, cet écrou 6 est bloqué en rotation dans son logement de réception 5, de sorte à empêcher sa rotation autour de son axe 6'.

La paroi centrale de l'orifice traversant 3 délimite le logement de réception 5 précité. L'orifice traversant 3 et le logement 5 ont le même axe 5'.

Le logement 5 de réception de l'écrou 6 est en particulier délimité, en partie supérieure (c'est-à-dire du côté de la surface 2' du support 2) par une surface 5a en forme générale de tronc de sphère, et en partie inférieure, par une surface 5b, également en tronc de sphère (cette surface 5b est destinée à venir se positionner du côté du matériau osseux de réception).
Les surfaces 5a et 5b sont concentriques ; elles sont centrées en un point situé sur l'axe 5' de l'orifice 3, dans l'épaisseur du support 2.
La partie supérieure 5a a un rayon supérieur à celui de la partie inférieure 5b ; de même, le diamètre de la bordure débouchante supérieure 3a de l'orifice traversant 3 est supérieur à celui de sa bordure débouchante inférieure 3b.

Plus précisément, la partie supérieure 5a du logement 5 est obtenue par un usinage approprié de la structure support 2 ; cette partie supérieure 5a est ainsi réalisée monobloc avec la structure support 2, ce qui optimise en particulier sa résistance aux forces de poussée.

La partie inférieure 5b du logement 5 est formée par la surface centrale d'un clip 7 rapporté, représenté en détails sur la figure 4. Ce clip 7 permet le verrouillage de l'écrou 6 dans son logement de réception 5, une fois convenablement rapporté.
Le clip 7 en question est en forme d'anneau circulaire ouvert, muni d'une fente 8, lui conférant une certaine élasticité radiale, en vue de son encastrement sur la structure support 2.
Sur son pourtour externe, ce clip 7 est pourvu d'une nervure ou bossage périphérique 9 apte à venir s'encastrer dans une gorge circulaire 10 ménagée convenablement au sein du logement de réception 5.

L'écrou 6, en forme d'anneau complet, comporte une surface ou contour périphérique 11, en forme générale de tronc de sphère, qui est complémentaire de la partie supérieure 5a du logement de réception 5.
Cette surface périphérique 11 est munie de cavités ou de réservations 12a (ici au nombre de trois), au sein desquelles viennent se loger des reliefs complémentaires 12b, formant des sortes de tenons, ménagés sur la partie supérieure 5a du logement 5. La coopération entre ces éléments 12a et 12b assure le blocage en rotation de l'écrou 6 autour de son axe 6' une fois convenablement en place dans son logement de réception 5 ; ces tenons 12b et réservations 12a sont structurés pour autoriser une certaine mobilité de l'écrou 6 au sein du logement 5, et en particulier pour permettre une certaine inclinaison de son axe 6' par rapport à l'axe 5' du logement 5, dans un domaine admissible prédéterminé.
L'orifice central 13 de l'écrou 6 est muni d'un filetage 14 (s'étendant autour de l'axe de symétrie 6').

La vis 4 se compose, de son côté, d'une tête de vis 15 et d'un corps de vis 16.

La tête de vis 15 comporte une partie supérieure 15a munie d'un filetage 17 destiné, en fin de vissage de la vis 4, à s'engager avec le filetage complémentaire 14 de l'écrou 6.
Cette tête de vis 15 comporte encore une partie inférieure 15b dont le contour a une forme générale de tronc de sphère, complémentaire de la partie inférieure 5b du logement de réception 5.

Le corps de vis 16 comporte quant à lui un filetage 18, s'étendant sur toute sa longueur, qui est conformé pour coopérer avec le matériau osseux de réception.

Les caractéristiques structurelles et dimensionnelles de ce corps de vis 16 avec le filetage 18 sont adaptées pour permettre son passage au travers de l'orifice fileté 13 de l'écrou 6, et plus généralement au travers de l'orifice 3 de la plaque support 2. En particulier, le diamètre externe du filetage 18 est prévu inférieur au diamètre externe du filetage 17 de la tête de vis 15, et aussi inférieur au diamètre de l'ouverture débouchante 3b de l'orifice 3.

Par ailleurs, le filetage de tête 17 est composé de 2 filets décalés de 1/2 tour, et dont le pas est identique à celui du filetage 14 de l'écrou 6 et à celui du filetage de corps 18.

La tête de vis 15 est conformée de sorte à venir se loger intégralement au sein de l'épaisseur de la structure support 2, de manière à ne pas faire saillie au-delà de la face supérieure 2', en fin de vissage, comme illustré sur la figure 1.

Pour cela, la hauteur de la tête de vis 15, c'est en dire la distance entre l'extrémité libre de sa partie supérieure 15a et l'extrémité inférieure de sa partie inférieure 15b, est inférieure ou égale à la hauteur de l'orifice traversant 3.

En pratique, tout d'abord, un écrou 6 est convenablement positionné au sein de chacun des logements de réception 5 de la structure support 2, cela de sorte que sa surface périphérique 11 vienne en regard de la partie supérieure 5a dudit logement de réception 5 et de sorte que ses réservations 12a reçoivent l'un des ergots complémentaires 12b.
Ensuite, ces écrous 6 sont emprisonnés dans leurs logements de réception 5 respectifs par la mise en place des clips de fermeture 7.

Cette structure support 2, équipée des écrous 6, est alors apte à être implantée par le praticien de sorte à traiter par exemple une fracture d'un patient.
Pour cela, il peut utiliser tout d'abord un canon de perçage, avant la mise en place des vis, pour initier des trous de positionnement dans le matériau osseux, au travers des orifices 3.
Ensuite, le praticien peut introduire et visser une vis de fixation 4 au travers de chacun des orifices traversants 3, ceci avec l'inclinaison adaptée par rapport à l'axe dudit orifice 3 (dans la limite du domaine d'inclinaison admissible). Cette possibilité d'inclinaison est en particulier liée au fait que l'écrou 6 dispose d'un degré de liberté dans son logement de réception 5, ledit écrou venant alors «automatiquement» s'aligner avec la vis de fixation 4. Le réglage angulaire de la vis 4 est possible à l'intérieur d'un volume conique d'un angle au sommet préférentiellement compris entre 20 et 30°, dont l'axe est confondu avec l'axe 5' de l'orifice traversant 3.

Le praticien poursuit le vissage de la vis 4, cela jusqu'à ce que la tête de vis 15 pénètre au sein de l'ensemble orifice 3/logement 5 (figures 1 à 3). Alors, comme on peut le voir sur la figure 3, le filetage 17 de la tête de vis 15 vient coopérer avec le filetage complémentaire 14 de l'écrou 6. En fin de vissage, la partie inférieure 15b de la tête de vis 15 vient prendre appui sur la surface complémentaire de la partie inférieure 5b du logement de réception 5 (on note que l'appui de la structure support 2 sur le matériau osseux de réception favorise le maintien du clip 7 en position) ; et cette coopération particulière tend à éloigner l'écrou 6 par rapport à la partie inférieure 5a du logement 5. Ce phénomène d'écartement conduit alors le contour d'écrou 11 à venir en appui forcé contre la partie supérieure complémentaire 5a du logement 5 ; également, la partie inférieure 15b de la tête de vis 15 vient en appui forcé contre la partie inférieure 5b complémentaire du logement 5.

Ce double appui forcé « en écartement », ou « en expansion », permet d'obtenir le verrouillage de la vis 4 au travers de son orifice 3 d'accueil ; l'intensité du verrouillage est notamment fonction de la force de vissage appliquée par le praticien dans cette phase finale de vissage.
Dans cette configuration finale, le contact des surfaces en tronc de sphère 5a et 5b du logement de réception 5 avec, simultanément, les surfaces complémentaires 11 et 15b de l'écrou 6 et de la tête de vis 15, permet d'obtenir un serrage efficace et de qualité, quelle que soit l'inclinaison de l'axe 4' de la vis 4 et de l'axe 6' de l'écrou 6 par rapport à l'axe 5' de l'ensemble orifice 3/logement 5 (dans le domaine d'inclinaison admissible).

Toujours dans cette configuration finale, la tête de vis 15 est complètement intégrée dans l'épaisseur de la structure support 2, sans aucune saillie particulière au-delà de la surface supérieure 2' du support 2.

En fonction des besoins, il suffira au praticien d'exercer une force de dévissage suffisante pour assurer le déverrouillage des vis en vue de leur enlèvement complet du matériau osseux de réception.

D'une manière générale, cette structure particulière de dispositif orthopédique implantable a l'intérêt de permettre l'obtention d'un verrouillage efficace de la vis au sein de l'ensemble orifice/logement de réception.
De plus, ce type de structure a l'intérêt d'être particulièrement compact et de pouvoir mettre en oeuvre des plaques supports dont l'épaisseur est limitée à quelques millimètres (de l'ordre de 2 à 3 mm d'épaisseur).

## Revendications

1. Dispositif orthopédique implantable comprenant :
- une structure support (2) délimitée par une face supérieure (2') et par une face inférieure (2"), cette dernière étant destinée à venir se positionner en regard du matériau osseux de réception, laquelle structure support (2) est munie d'au moins un logement (5) et d'au moins un orifice (3) passant au travers dudit logement (5), ledit logement (5) et ledit orifice (3) ayant le même axe (5'),
- au moins un écrou (6) muni d'un filetage interne (14), lequel écrou (6) est conformé pour être emprisonné au sein de l'un desdits logements (5), et
- au moins une vis de fixation (4) composée d'une tête de vis (15) et d'un corps de vis (16), laquelle tête de vis (15) est munie d'un filetage (17) apte à coopérer avec ledit filetage d'écrou (14) et lequel corps de vis (16) est muni d'un filetage (18) apte à coopérer avec le matériau osseux de réception,
lequel écrou (6) est bloqué en rotation dans ledit logement de réception (5) de sorte à empêcher sa rotation autour de son axe (6'), et lequel écrou (6) comporte au moins un degré de liberté dans ledit logement de réception (5), selon un domaine d'inclinaison prédéterminé admissible, pour permettre une inclinaison de son axe (6') par rapport à l'axe (5') dudit logement de réception (5),
laquelle vis de fixation (4) est destinée à être vissée au travers l'un desdits orifices (3) et de son écrou (6) associé, cela selon un angle sélectionné dans le domaine d'inclinaison admissible,
**caractérisé en ce que** ledit logement (5) comporte une partie supérieure (5a) située du côté de la face supérieure (2') de la structure support (2), et une partie inférieure (5b), située du côté de la face inférieure (2") de ladite structure support (2), toutes deux en forme générale de tronc de sphère, concentriques l'une par rapport à l'autre,
**en ce que** ledit écrou (6) comporte un contour périphérique (11) en forme de tronc de sphère, complémentaire de ladite partie supérieure en tronc de sphère (5a) du logement de réception (5),
et **en ce que** ladite tête de vis (15) comporte une partie supérieure (15a) munie dudit filetage (17) et une partie inférieure (15b) dont le contour a une forme générale en tronc de sphère, complémentaire de ladite partie inférieure en tronc de sphère (5b) du logement de réception (5),
lesquelles parties supérieure (15a) et inférieure (15b) de ladite tête de vis (15) sont destinées à coopérer, lors de la phase finale de vissage de ladite vis (4), respectivement avec le filetage (14) dudit écrou (6) par le biais de son filetage complémentaire (17) et avec ladite partie inférieure (5b) du logement de réception (5), cela de sorte à tendre à éloigner ledit écrou (6) par rapport à ladite partie inférieure (5b) du logement de réception (5), et provoquer ainsi un appui forcé du contour périphérique (11) dudit écrou (6) contre ladite partie supérieure complémentaire (5a) du logement de réception (5) et un appui forcé de la partie inférieure (15b) de ladite tête de vis (15) contre ladite partie inférieure complémentaire (5b) du logement de réception (5), cela pour obtenir le verrouillage de ladite vis (4) en phase finale de vissage.

2. Dispositif implantable selon la revendication 1, **caractérisé en ce que** les parties en tronc de sphère supérieure (5a) et/ou inférieure (5b) du logement (5) sont constituées par la surface centrale d'un clip rapporté (7), destiné à fermer partiellement ledit logement de réception (5) pour assurer le maintien de l'écrou (6).

3. Dispositif implantable selon la revendication 2, **caractérisé en ce que** les parties en tronc de sphère supérieure (5a) et inférieure (5b) du logement (5) de réception de l'écrou (6) sont constituées respectivement par une surface monobloc de la structure support (2) et par la surface centrale du clip rapporté (7).

4. Dispositif implantable selon l'une quelconque des revendications 2 ou 3, **caractérisé en ce que** le clip (7) de maintien d'écrou (6) a une forme générale d'anneau ouvert, lequel anneau (7) comporte une nervure d'encliquetage (9) apte à coopérer avec une gorge (10) de forme appropriée aménagée dans le logement de réception (5).

5. Dispositif implantable selon l'une quelconque des revendications 1 à 4, **caractérisé en ce que** l'encombrement général de la tête de vis (15) correspond au diamètre de la surface centrale (13) de l'écrou (6), ledit encombrement de tête de vis (15) étant encore inférieur à celui de la partie supérieure (5a) du logement de réception (5).

6. Dispositif implantable selon l'une quelconque des revendications 1 à 5, **caractérisé en ce que** la hauteur de la tête de vis (15), entre son extrémité libre et l'extrémité inférieure de sa partie inférieure en tronc de sphère (15b), est inférieure ou égale à la hauteur de l'orifice traversant (3) associé.

7. Dispositif implantable selon l'une quelconque des revendications 1 à 6, **caractérisé en ce que** le logement (5) de réception de l'écrou (6) comporte une partie supérieure en tronc de sphère (5a) dont le rayon est plus grand que celui de la partie inférieure en tronc de sphère (5b).

8. Dispositif implantable selon l'une quelconque des revendications 1 à 7, **caractérisé en ce qu'**il comprend au moins un relief (12b) et une réservation complémentaire (12a), l'un étant ménagé au niveau du logement de réception (5) et l'autre étant ménagé au niveau au niveau du contour périphérique (11) de l'écrou (6), ledit relief (12b) et ladite réservation (12a) étant positionnés et conformés de manière à former les moyens (12) de blocage de l'écrou (6) en rotation dans son logement de réception (5).

9. Dispositif implantable selon l'une quelconque des revendications 1 à 8, **caractérisé en ce que** le diamètre externe du filetage de corps (18) de la vis (4) est inférieur au diamètre externe du filetage de tête (17) de ladite vis (4), et **en ce que** ledit filetage de tête (17) est composé de n filets, n étant supérieur ou égal à 2, décalés de 1/n tour, dont le pas correspond à celui du filetage (14) de l'écrou (6) et à celui dudit filetage de corps (18).

## Patentansprüche

1. Implantierbare orthopädische Vorrichtung mit
- einer Trägerstruktur (2), die durch eine Oberseite (2') und eine Unterseite (2") begrenzt ist, wobei letztere dazu bestimmt ist, gegenüber dem aufnehmenden Knochenmaterial positioniert zu werden, wobei die Trägerstruktur (2) mit wenigstens einem Aufnahmeraum (5) und wenigstens einer den Aufnahmeraum (5) durchquerenden Öffnung (3) versehen ist, wobei der Aufnahmeraum (5) und die Öffnung (3) dieselbe Achse (5') haben,
- wenigstens einer mit einem Innengewinde (14) versehenen Mutter (6), die dazu ausgebildet ist, im Inneren eines der besagten Aufnahmeräume (5) eingeschlossen zu sein, und
- wenigstens einer Fixierschraube (4), die aus einem Schraubenkopf (15) und einem Schraubenkörper (16) besteht, wobei der Schraubenkopf (15) mit einem Gewinde (17) versehen ist, das geeignet ist, mit dem Gewinde (14) der Mutter zusammenzuwirken, und wobei der Schraubenkörper (16) mit einem Gewinde (18) versehen ist, das geeignet ist, mit dem aufnehmenden Knochenmaterial zusammenzuwirken,
wobei die Mutter (6) im Aufnahmeraum (5) gegen Drehen gesichert ist, um ein Drehen derselben um deren Achse (6') zu verhindern, und wobei die Mutter (6) im Aufnahmeraum (5) wenigstens einen Freiheitsgrad gemäß einem zulässigen vorbestimmten Neigungsbereich aufweist, um eine Neigung von deren Achse (6') gegenüber der Achse (5') des Aufnahmeraums (5) zu ermöglichen, wobei die Fixierschraube (4) dazu bestimmt ist, durch eine der Öffnungen (3) und durch die zugehörige Mutter (6) hindurch geschraubt zu werden, und zwar mit einer innerhalb des zulässigen Neigungsbereichs liegenden Neigung,
dadurch gekenntzeichnet, daß der Aufnahmeraum (5) einen auf der Seite der Oberseite (2') der Trägerstruktur (2) liegenden oberen Teil (5a) und einen auf der Seite der Unterseite (2") der Trägerstruktur (2) liegenden unteren Teil (5b) aufweist, beide mit der allgemeinen Form einer Kugelschicht und zueinander konzentrisch,
daß die Mutter (6) eine Umfangskontur (11) in Form einer Kugelschicht aufweist, die zum oberen Teil (5a) des Aufnahmeraums (5) in Form einer Kugelschicht komplementär ist,
und daß der Schraubenkopf (15) einen mit dem besagten Gewinde (17) versehenen oberen Teil (15a) und einen unteren Teil (15b), dessen Kontur die zum unteren Teil (5b) des Aufnahmeraums (5) in Form einer Kugelschicht komplementäre allgemeine Form einer Kugelschicht hat, aufweist,
wobei der obere (15a) und der untere (15b) Teil des Schraubenkopfs (15) dazu bestimmt sind, am Ende des Schraubvorgangs der Schraube (4) mit dem Gewinde (14) der Mutter (6) mittels dessen komplementäre Gewindes (17) bzw. mit dem unteren Teil (5b) des Aufnahmeraums (5) zusammenzuwirken, und dies derart, daß die Mutter (6) dazu neigt, vom unteren Teil (5b) des Aufnahmeraums (5) wegbewegt zu werden und daß dadurch ein erzwungener Andruck der Umfangskontur (11) der Mutter (6) an den komplementären oberen Teil (5a) des Aufnahmeraums (5) und ein erzwungener Andruck des unteren Teils (15b) des Schraubenkopfs (15) an den komplementäre unteren Teil (5b) des Aufnahmeraums (5) hervorgerufen wird, und zwar um die Verriegelung des Schraubenkopfs (4) in der Endphase des Schraubens zu erhalten.

2. Implantierbare Vorrichtung gemäß Anspruch 1, **dadurch gekennzeichnet, daß** das obere (5a) und/oder das untere (5b) Teil des Aufnahmeraums (5) in Form einer Kugelschicht durch die zentrale Oberfläche eines hinztigefügten Klipses (7) gebildet sind, der dazu bestimmt ist, den Aufnahmeraum (5) teilweise zu erschließen, um das Zurückhalten der Mutter (6) sicherzustellen.

3. Implantierbare Vorrichtung gemäß Anspruch 2, **dadurch gekennzeichnet, daß** das obere (5a) und das untere (5b) Teil des Raums (5) in Form einer Kugelschicht zur Aufnahme der Mutter (6) durch eine einstückige Oberfläche der Trägerstruktur (2) bzw. durch die zentrale Oberfläche eines hinzugefügten Klipses (7) gebildet sind.

4. Implantierbare Vorrichtung gemäß einem der Ansprüche 2 oder 3, **dadurch gekennzeichnet, daß** der Klips (7) zum Zurückhalten der Mutter (6) die allgemeine Form eines offenen Rings aufweist, wobei der Ring (7) eine Einrastrippe (9) aufweist, die geeignet ist, mit einer im Aufnahmeraum (5) eingebrachten Rille (10) geeigneter Form zusammenzuwirken.

5. Implantierbare Vorrichtung gemäß einem der Ansprüche 1 bis 4, dadurch gekenntzeichnet, daß die Gesamtabmessungen des Schraubenkopfes (15) dem Durchmesser der zentralen Oberfläche (13) der Mutter (6) entsprechen, wobei die Gesamtabmessungen des Schraubenkopfes (15) noch kleiner als jene des oberen Teils (5a) des Aufnahmeraums (5) sind.

6. Implantierbare Vorrichtung gemäß einem der Ansprüche 1 bis 5, **dadurch gekennzeichnet, daß** die Höhe des Schraubenkopfes (15) zwischen seinem freien Ende und dem unteren Ende seines unteren Teils (15b) in Form einer Kugelschicht kleiner als oder gleich der Höhe der zugehörigen durchgehenden Öffnung (3) ist.

7. Implantierbare Vorrichtung gemäß einem der Ansprüche 1 bis 6, dadurch gekenntzeichnet, daß der Raum (5) zur Aufnahme der Mutter (6) einen oberen Teil (5a) in Form einer Kugelschicht aufweist, deren Radius größer ist als jener des unteren Teils (5b) in Form einer Kugelschicht.

8. Implantierbare Vorrichtung gemäß einem der Ansprüche 1 bis 7, dadurch gelcennzeichnet, daß sie wenigstens ein Relief (12b) und eine komplementäre Ausnehmung (12a) aufweist, wobei das eine im Aufnahmeraum (5) ausgebildet ist und das andere an der Umfangskontur (11) der Mutter (6) ausgebildet ist, wobei das Relief (12b) und die Ausnehmung (12a) derart angeordnet und ausgebildet sind, daß sie im Aufnahmeraum (5) die Mittel (12) zum Sichern der Mutter (6) gegen Drehen bilden.

9. Implantierbare Vorrichtung gemäß einem der Ansprüche 1 bis 8, **dadurch gekennzeichnet, daß** der Außendurchmesser des Gewindes (18) des Körpers der Schraube (4) kleiner als der Außendurchmesser des Gewindes (17) des Kopfes der Schraube (4) ist und daß das Gewinde (17) des Kopfes n Gänge aufweist, wobei n größer als oder gleich 2 ist, die um 1/n Drehung versetzt sind, deren Gewindesteigung jener des Gewindes (14) der Mutter (6) und jenem des Gewindes (18) des Körpers entspricht.

## Claims

1. Implantable orthopedic device comprising:
- a support structure (2) delimited by an upper face (2') and a lower face (2"), the latter being intended to come opposite to the receiving bone material, said support structure (2) being provided with at least one housing (5) and at least one orifice (3) passing through said housing (5), said housing (5) and said orifice (3) having the same axis (5'),
- at least one nut (6) provided with an internal thread (14), said nut (6) being shaped so as to be caught within one of said housings (5), and
- at least one fixing screw (4) consisting of a screw head (15) and a screw body (16), said screw head (15) being provided with a thread (17) able to cooperate with said nut thread (14), and said screw body (16) being provided with a thread (18) able to cooperate with the receiving bone material,
said nut (6) being locked in rotation within said receiving housing (5) so as to be prevented from rotating around its axis (6'), and said nut (6) having at least one freedom degree within said receiving housing (5), according to a predetermined acceptable tilting domain, so as to enable a tilting of its axis (6') relative to the axis (5') of said receiving housing (5),
said fixing screw (4) being intended to be screwed within one of said orifices (3) and the associated nut (6) thereof, according to a selected angle in the acceptable tilting domain,
**characterized in that** said housing (5) comprises an upper part (5a) located on the upper face (2') side of the support structure (2) and a lower part (5b) located on the lower face (2") side of said support structure (2), both parts being generally truncated sphere shaped and concentric to each other,
**in that** said nut (6) has a truncated sphere shaped peripheral contour (11) mating with said truncated sphere shaped upper part (5a) of the receiving housing (5),
and **in that** said screw head (15) comprises an upper part (15a) provided with said thread (17) and a lower part (15b) whose contour has a generally truncated sphere shape mating with said truncated sphere shaped lower part (5b) of the receiving housing (5),
said upper (15a) and lower (15b) parts of said screw head (15) being intended to cooperate, during the final screwing step of said screw (4), with the thread (14) of said nut (6) through the mating thread (17) thereof and with said lower part (5b) of the receiving housing (5), respectively, so as to tend to separate said nut (6) from said lower part (5b) of the receiving housing (5), and then entail a forced rest of the peripheral contour (11) of said nut (6) against said mating upper part (5a) of the receiving housing (5) and a forced rest of the lower part (15b) of said screw head (15) against said mating lower part (5b) of the receiving housing (5), in order to obtain the locking of said screw (4) during the final screwing step.

2. Implantable device according to claim 1, **characterized in that** truncated sphere shaped upper (5a) and/or lower (5b) parts of the housing (5) are constituted by the central surface of an add-on clip (7) intended to partially close said receiving housing (5) in order to ensure the stay-on of the nut (6).

3. Implantable device according to claim 2, **characterized in that** truncated sphere shaped upper (5a) and lower (5b) parts of the receiving housing (5) of the nut (6) are constituted by a surface integral with the support structure (2) and by the central surface of the add-on clip (7), respectively.

4. Implantable device according to any one of claims 2 or 3, **characterized in that** the clip (7) for the stay-on of the nut (6) is in the general form of an open ring, said ring (7) comprises a snapping rib (9) able to cooperate with a suitably shaped groove (10) arranged in the receiving housing (5).

5. Implantable device according to any one of claims 1 to 4, **characterized in that** overall dimensions of the screw head (15) correspond to the diameter of the central surface (13) of the nut (6), said dimensions of the screw head (15) being yet smaller than those of the upper part (5a) of the receiving housing (5).

6. Implantable device according to any one of claims 1 to 5, **characterized in that** the height of the screw head (15), between its free end and the lower end of its truncated sphere shaped lower part (15b), is lower or equal to the height of the associated through-orifice (3).

7. Implantable device according to any one of claims 1 to 6, **characterized in that** the receiving housing (5) of the nut (6) comprises a truncated sphere shaped upper part (5a) whose radius is greater than that of truncated sphere shaped lower part (5b).

8. Implantable device according to any one of claims 1 to 7, **characterized in that** it comprises at least one embossment (12b) and a mating recess (12a), one being arranged at the receiving housing (5) and the other being arranged at the peripheral contour (11) of the nut (6), said embossment (12b) and said recess (12a) being positioned and shaped so as to form the means for locking the nut (6) in rotation within its receiving housing (5).

9. Implantable device according to any one of claims 1 to 8, **characterized in that** the external diameter of the body thread (18) of the screw (4) is smaller than the external diameter of the head thread (17) of said screw (4), and **in that** said head thread (17) consists of n threads, n being higher or equal to 2, which are shifted by 1/n turn and whose pitch corresponds to that of the thread (14) of the nut (6) and to that of said body thread (18).
